## Europäisches Patentamt

## European Patent Office

## Office européen des brevets

(19)

(11) Veröffentlichungsnummer: **0 056 595**
**B1**

(12) # EUROPÄISCHE PATENTSCHRIFT

(45) Veröffentlichungstag der Patentschrift:
31.10.84

(21) Anmeldenummer: 82100129.4

(22) Anmeldetag: 11.01.82

(51) Int. Cl.³: **A 61 K 7/06,** A 61 K 7/08,
A 61 K 7/11

(54) Haarbehandlungsmittel und Verfahren zur Verbesserung des Zustandes von Haaren.

(30) Priorität: 15.01.81 DE 3101011

(43) Veröffentlichungstag der Anmeldung:
28.07.82 Patentblatt 82/30

(45) Bekanntmachung des Hinweises auf die Patenterteilung:
31.10.84 Patentblatt 84/44

(84) Benannte Vertragsstaaten
AT DE FR GB IT NL

(56) Entgegenhaltungen:
BE - A - 669 472
DE - B - 1 007 957
US - A - 1 811 809

SEIFEN-ÖLE-FETTE-WACHSE, Band 99, No. 5, 01. März 1973 AUGSBURG (DE) R. LEUTERITZ: "Haarwässer und reinigende Haarpflege", Seite 125

(73) Patentinhaber: Wella Aktiengesellschaft, Berliner Allee 65, D-6100 Darmstadt (DE)

(72) Erfinder: Konrad, Eugen, Mecklenburger Strasse 101, D-6100 Darmstadt (DE)
Erfinder: Mager, Herbert, Dr., Beaumont 5, CH-1700 Fribourg (CH)
Erfinder: Hoch, Dietrich, Ringstrasse 48, D-6102 Pfungstadt (DE)

## Beschreibung

Durch öfteres Bleichen, Dauerwellen und Färben, aber auch durch häufiges Waschen der Haare mit entfettenden Tensiden, kommt es zu einer Schädigung der Haarstruktur. Das Haar wird spröde, und es verliert seinen Glanz. Weiterhin lädt sich das Haar beim Kämmen elektrostatisch auf, und die aufgerauhte Haaroberfläche verursacht Verfilzungen sowie Verknotungen des Haares. Hierdurch wird das Kämmen sehr erschwert.

Haarbehandlungsmittel mit einer kämmbarkeitsverbessernden und pflegenden Wirkung haben daher eine erhebliche Bedeutung erlangt. Derartige Mittel werden beispielsweise häufig in Form einer klaren Haarpflegespülung oder auch in Emulsionsform, als sogenannte Creme-Rinses, nach der Haarwäsche im noch nassen Haar verteilt, einige Minuten bis eine Stunde einwirken gelassen und dann mit Wasser ausgespült.

Als Wirkstoffe zur Verbesserung der Haarstruktur werden hauptsächlich kationische Tenside, insbesondere quaternäre Ammoniumverbindungen, wie Cetyltrimethylammoniumchlorid, in Kombination mit verschiedenen wachsartigen Zusätzen, wie zum Beispiel Vaseline, Fettalkoholen und Fettsäureestern, eingesetzt.

Haarbehandlungsmittel auf der Basis der vorstehenden konditionierenden Wirkstoffe zeigen jedoch nur bei der Behandlung von trockenem und porösem Haar zufriedenstellende Ergebnisse. Für die Behandlung von rasch nachfettendem Haar sind sie weniger gut geeignet, da durch ihre Anwendung die natürliche Nachfettung der Haare noch verstärkt wird, wodurch sich wiederum die Haltbarkeit der Frisur verschlechtert.

Die Ursachen der starken Nachfettung der Haare sind zum einen die nach dem Ausspülen im Haar verbleibenden Reste des Haarbehandlungsmittels, zum anderen die in diesen Mitteln enthaltenen kationischen Emulgatoren. Die vom Haar adsorbierten kationischen Emulgatoren bewirken eine Hydrophobierung der Haaroberfläche, wodurch sich die Absonderungen der Talgdrüsen rascher im Haar verteilen können. Außerdem lassen sich kationische Emulgatoren wegen ihrer Unverträglichkeit mit anionischen Tensiden in Haarbehandlungsmitteln mit einem Gehalt an diesen Tensiden, wie zum Beispiel in vielen Shampoos oder Haarfärbemitteln, nicht einarbeiten.

Es sind auch bereits Haarbehandlungsmittel beschrieben worden, welche dem Zweck dienen, den Haarausfall zu verhindern und das Haarwachstum zu fördern. Die Mittel können auch einen Gehalt an Betain und gleichzeitig an den Aminosäuren Glykoll und Cystin aufweisen. Sie sollen mehrmals täglich auf die Kopfhaut aufgebracht und leicht einmassiert werden (vgl. DE-B-1 007 957).

Demgemäß besteht die Aufgabe der Erfindung darin, ein Haarbehandlungsmittel und ein Haarbehandlungsverfahren auf der Basis besser geeigneter haarkonditionierender Wirkstoffe zur Verfügung zu stellen und dadurch die vorstehend geschilderten Nachteile zu vermeiden.

Hierzu wurde nun gefunden, daß Haarbehandlungsmittel, dadurch gekennzeichnet, daß sie eine Kombination von

a) 0,1 bis 25,0 Gew.-% Betain
$$\left[\begin{array}{l} = \text{Trimethylammonioacetat der Formel} \\ (CH_3)_3N^{\oplus}{-}CH_2{-}COO^{\ominus} \end{array}\right]$$
und

b) 0,1 bis 10,0 Gew.-% mindestens einer von Aminogruppen freien aliphatischen organischen Säure

enthalten, die gestellte Aufgabe in hervorragender Weise erfüllen.

Während bei Haarbehandlungsmitteln auf der alleinigen Basis von Betain oder auf der alleinigen Basis einer aliphatischen organischen Säure keine merklichen haarkonditionierenden Eigenschaften festzustellen sind, zeigen die erfindungsgemäßen Haarbehandlungsmittel auf der Basis einer synergistischen Kombination von Betain und einer von Aminogruppen freien aliphatischen organischen Säure eine gute kämmbarkeitsverbessernde Wirkung, ohne dabei das Haar zu belasten. Sie wirken weiterhin adstringierend und entwirrend auf das Haar, glätten die Haaroberfläche und verbessern den Griff des Haares.

Zur Herstellung der Mittel gemäß der vorliegenden Anmeldung kann man neben reinem Betain selbstverständlich das Betain auch in beliebiger anderer handelsüblicher Form, beispielsweise als Betainmonohydrat, verwenden.

Beispiele für geeignete, in den hier beschriebenen Mitteln enthaltene von Aminogruppen freie aliphatische organische Säuren sind insbesondere wasser- oder wasser-alkohollösliche aliphatische organische Säuren, wie Zitronensäure, Weinsäure, Milchsäure, Pimelinsäure und Glyoxylsäure.

Die Mittel gemäß der Erfindung sollen das Betain insbesondere in einer Konzentration von 3,0 bis 15,0 Gew.-% enthalten, während die von Aminogruppen freien aliphatischen organischen Säuren — von denen die Zitronensäure bevorzugt ist — allein oder im Gemisch miteinander, insbesondere in einer Menge von 0,1 bis 5,0 Gew.-%, enthalten sind.

Die in der Anmeldung beschriebenen Haarbehandlungsmittel können in einer beliebigen für die Haarbehandlung geeigneten Zubereitungsform, wie zum Beispiel in Form einer Lotion, Emulsion oder eines Gels, vorliegen. Bevorzugte Zubereitungen sind Haarspülungen, Haarpflegeemulsionen, Haarkurpackungen, Haarfestiger oder Shampoos. Die erfindungsgemäßen Haarbehandlungsmittel können aber auch als Haarfärbemittel, Haartönungsmittel oder als Fixiermittel für die Haarverformung vorliegen.

Es handelt sich dabei um Zubereitungen, die je nach ihrem Anwendungszweck für kürzere oder längere Zeit auf dem Haar verbleiben. Durch ihren Gehalt an der beschriebenen erfindungsgemäßen Wirkstoffkombination wird gleichzeitig eine Konditionierung des behandelten Haares bewirkt. Besonders bevorzugt sind jedoch Zubereitungen, die hauptsächlich oder ausschließlich dem Ziel einer Verbesserung des Zustandes der Haarstruktur dienen.

Die Zusammensetzung dieser kosmetischen Zubereitungen stellt eine Mischung der konditionierend wirkenden Kombination gemäß vorliegender Erfindung mit den für Haarbehandlungsmittel üblichen weiteren Bestandteilen dar.

Als übliche Bestandteile von Haarbehandlungsmitteln kommen insbesondere Wasser, Alkohole, wie zum Beispiel Ethanol, n-Propanol, i-Propanol, mehrwertige Alkohole, wie Glycerin und Propylenglykol, anionische, kationische, amphotere oder nichtionogene Tenside, wie Fettalkoholsulfate, Fettalkoholethersulfate, Alkylsulfonate, Alkylbenzolsulfonate, Fettsäurealkyltrimethylammoniumsalze, oxethylierte Fettalkohole, oxethylierte Nonylphenole, Fettsäurealkanolamide, weiterhin natürliche, modifizierte natürliche oder synthetische Polymere, wie zum Beispiel Schellack, Alginate, Gelatine, Pektine, Cellulosederivate, Chitosan, Polyvinylpyrrolidon, Polyvinylacetat, Acrylsäure- oder Methacrylsäurepolymerisate, basische Polymerisate von Estern aus Acrylsäure oder Methacrylsäure mit Aminoalkoholen beziehungsweise die Salze oder Quaternisierungsprodukte dieser basischen Polymerisate, Polyacrylnitril sowie Copolymerisate aus derartigen Verbindungen, wie Polyvinylpyrrolidon-vinylacetat, ferner Verdicker, wie Fettalkohole, Fettsäureester, Stärke, Cellulosederivate, flüssige und feste Paraffine, Isoparaffine, Vaseline, Wollwachs und Fettsäuren sowie außerdem Pflegestoffe wie Lanolinderivate, Cholesterin und Pantothensäure, weiterhin Farbstoffe, Pigmente, Parfümöle, Antioxidantien, Konservierungsmittel, wie zum Beispiel Formaldehyd und Salicylsäure, in Betracht.

Bei dem erfindungsgemäßen Verfahren zur Verbesserung des Zustandes von Haaren wird das Haar mit einem Haarbehandlungsmittel, insbesondere mit einer Haarpflegespülung, dadurch gekennzeichnet, daß es eine Kombination von

a)   0,1 bis 25 Gew.-% Betain und
b)   0,1 bis 10,0 Gew.-% mindestens einer von Aminogruppen freien aliphatischen organischen Säure

enthält, bei einer Temperatur von etwa 15 bis 60°C in Kontakt gebracht.

Die in den erfindungsgemäßen Haarbehandlungsmitteln vorliegenden haarkonditionierend wirksamen Betainsalze — zum Beispiel das Betaincitrat — sind gut in Wasser oder in Wasser-Alkohol-Gemischen löslich und physiologisch, auch bei sehr hoher Konzentration, völlig unbedenklich.

Außerdem hat sich überraschend gezeigt, daß dünnflüssige Emulsionen mit einem Gehalt an 10 Gew.-% Betain und 2 Gew.-% Zitronensäure, trotz dieses hohen Elektrolytgehaltes, bei geeigneter Wahl der Wachskomponenten und der Emulgatoren problemlos herstellbar sind.

Darüber hinaus kann die hier beschriebene synergistisch wirksame Kombination gemäß der vorliegenden Erfindung ohne Ausfällung in Präparate mit einem Gehalt an anionischen, kationischen, nichtionogenen oder amphoteren Tensiden eingearbeitet werden.

Die in den Haarbehandlungsmitteln enthaltene Kombination von Betain und einer von Aminogruppen freien aliphatischen organischen Säure wirkt in diesen Zubereitungen weiterhin antioxidativ und als Puffer. Darüber hinaus ist mit Haarfestigern, die einen Gehalt an mindestens 1 Gew.-% Betain und mindestens 0,2 Gew.-% einer von Aminogruppen freien aliphatischen organischen Säure aufweisen, auch ohne Zusatz eines Harzes eine merkliche haarfestigende Wirkung zu erzielen. In Haarfestigern kann also die erfindungsgemäße Wirkstoffkombination das üblicherweise erforderliche Harz ersetzen.

Ein wichtiger Vorteil der erfindungsgemäßen Haarbehandlungsmittel ist schließlich ihre gegenüber Mitteln auf der Basis von üblichen kationischen haarkonditionierenden Wirkstoffen, wie zum Beispiel Fettsäurealkyltrimethylammoniumsalzen, wesentlich bessere Augen- und Hautverträglichkeit.

Die nachstehenden Beispiele sollen den Gegenstand der Erfindung näher erläutern.

Beispiele

Beispiel 1

Haarpflegespülung

  10,0 g   Betainmonohydrat
   2,0 g   Weinsäure und/oder Zitronensäure, wasserfrei
   0,1 g   Formaldehyd, 35%ig
  87,9 g   Wasser, vollentsalzt
 100,0 g

Diese Haarpflegespülung wird nach der Haarwäsche auf dem handtuchtrockenen porösen Haar gut verteilt, einige Minuten einwirken gelassen und anschließend mit Wasser ausgewaschen. Das Haar ist deutlich weniger verfilzt und besser kämmbar.

Beispiel 2

Haarpflegespülung

  10,0 g   Betainmonohydrat
   2,0 g   Zitronensäure, wasserfrei
   0,1 g   Formaldehyd, 35%ig
   7,0 g   Isoparaffin
  80,9 g   Wasser, vollentsalzt
 100,0 g

Diese aus zwei flüssigen Phasen bestehende Haarpflegespülung wird kurz vor dem Gebrauch geschüttelt und dann wie in Beispiel 1 auf stumpfem und porösem Haar angewendet. Das Haar ist nach der Behandlung leicht kämmbar und geschmeidig.

### Beispiel 3

#### Haarpflegeemulsion

10,0 g  Betainmonohydrat
5,0 g  Zitronensäure, wasserfrei
2,6 g  Gemisch aus 50% Cetylalkohol und 50% Stearylalkohol, Erstarrungspunkt: 48—52°C
0,5 g  Gemisch aus 50% Natriumcetylsulfat und 50% Natriumstearylsulfat
1,5 g  Wollwachsalkohol
0,9 g  Glycerin-mono-stearatpalmitat (85% 1-Monoglyceride; 10% 2-Monoglyceride; HLB-Wert: 4,5; Säurezahl: max. 1,5; Verseifungszahl: 163—170; Jodzahl: max 3)
0,2 g  p-Hydroxybenzoesäuremethylester
0,2 g  Salicylsäure
0,5 g  Parfümöl
78,6 g  Wasser
100,0 g

Die Haarpflegeemulsion wird nach der Haarwäsche in einem stark verfilzten, sehr porösen Haar gleichmäßig verteilt. Bereits beim Auftragen ist eine Entwirrung der Haare festzustellen. Nach kurzer Einwirkungszeit wird das Haar mit warmem Wasser ausgespült. Durch diese Behandlung wird ein sehr glatter, kosmetisch angenehmer Griff des Haares und eine sehr gute Naßkämmbarkeit erreicht.

### Beispiel 4

#### Haarfestiger

0,5 g  Betainmonohydrat
0,1 g  Zitronensäure, wasserfrei
40,0 g  Isopropanol
2,0 g  Copolymer aus 60% Vinylpyrrolidon und 40% Vinylacetat, in Pulverform
0,5 g  Parfümöl
56,9 g  Wasser, vollentsalzt
100,0 g

Dieser Haarfestiger wird nach der Haarwäsche gleichmäßig auf das handtuchtrockene Haar aufgetragen. Anschließend wird das Haar auf Wasserwellwickler aufgerollt. Nach der Trocknung des Haares, die zweckmäßigerweise mit Hilfe einer Trockenhaube bei etwa 40—60°C erfolgen kann, ist eine gute Haarfestigung, eine verbesserte Haltbarkeit der Frisur und ein sehr geschmeidiger Griff des Haares festzustellen.

### Beispiel 5

#### Haarfarbfestiger

0,5 g  Betainmonohydrat
0,1 g  Zitronensäure, wasserfrei
0,1 g  Farbstoff Acid Brown 4 (Color Index Nr. 14 805)
2,0 g  Copolymer aus 60% Vinylpyrrolidon und 40% Vinylacetat, in Pulverform
40,0 g  Isopropanol
0,5 g  Parfümöl
56,8 g  Wasser, vollentsalzt
100,0 g

Dieser Haarfarbfestiger wird nach der Haarwäsche gleichmäßig auf handtuchtrockene blonde menschliche Haare aufgetragen. Anschließend werden die Haare auf Wasserwellwickler aufgerollt. Nach dem Trocknen des Haares ist eine gute Haarfestigung, eine verbesserte Haltbarkeit der Frisur und ein sehr geschmeidiger Griff des Haares festzustellen. Die Haare weisen außerdem eine rötlich-blonde Färbung auf.

### Beispiel 6

#### Haarfestiger

2,5 g  Betain
0,5 g  Zitronensäure, wasserfrei
40,0 g  Isopropanol
0,5 g  Parfümöl
56,5 g  Wasser, vollentsalzt
100,0 g

Dieser Haarfestiger wird nach der Haarwäsche gleichmäßig auf das handtuchtrockene Haar aufgetragen. Anschließend wird das Haar auf Wasserwellwickler aufgerollt. Nach dem Trocknen des Haares ist — obwohl der Haarfestiger kein Harz enthält — eine gute Haarfestigung und eine verbesserte Haltbarkeit der Frisur festzustellen.

### Beispiel 7

#### Shampoo

8,0 g  Betainmonohydrat
2,0 g  Zitronensäure, wasserfrei
0,1 g  Formaldehyd, 35%ig
40,0 g  Laurylalkohol-diglykolethersulfat, Natriumsalz (28%ige wäßrige Lösung)
3,5 g  Natriumchlorid
46,4 g  Wasser
100,0 g

Menschliche Haare, die mit einem Shampoo der obigen Zusammensetzung gewaschen und anschließend mit Wasser gespült wurden, zeigen eine sehr gute Naßkämmbarkeit. Außerdem wird durch das in diesem Shampoo enthaltene Betain der pH-Wert des Shampoos stabilisiert und einer raschen Austrocknung der Kopfhaut entgegenwirkt.

Sämtliche in der Anmeldung angegebenen Prozentzahlen stellen Gewichtsprozente dar.

**Patentansprüche**

1. Haarbehandlungsmittel, dadurch gekennzeichnet, daß es eine Kombination von

a) 0,1 bis 25,0 Gew.-% Betain und
b) 0,1 bis 10,0 Gew.-% mindestens einer von Aminogruppen freien aliphatischen organischen Säure

enthält.

2. Haarbehandlungsmittel nach Anspruch 1, dadurch gekennzeichnet, daß das Betain in einer Menge von 3,0 bis 15,0 Gew.-% enthalten ist.

3. Haarbehandlungsmittel nach Anspruch 1 und 2, dadurch gekennzeichnet, daß die von Aminogruppen freie aliphatische organische Säure ausgewählt ist aus Milchsäure, Weinsäure, Pimelinsäure, Glyoxylsäure und Zitronensäure.

4. Haarbehandlungsmittel nach Anspruch 1 bis 3, dadurch gekennzeichnet, daß es eine Haarspülung, eine Haarpflegeemulsion, ein Haarfestiger oder ein Shampoo ist.

5. Haarbehandlungsmittel nach Anspruch 1 bis 4, dadurch gekennzeichnet, daß es

a) 3,0 bis 15,0 Gew.-% Betain und
b) 0,1 bis 5,0 Gew.-% Zitronensäure

enthält.

6. Verfahren zur Verbesserung des Zustandes von Haaren, dadurch gekennzeichnet, daß das Haar mit einem Haarbehandlungsmittel nach einem der Ansprüche 1 bis 5 bei einer Temperatur von etwa 15 bis 60° C in Kontakt gebracht wird.

**Claims**

1. Hair treatment medium, characterised in that it contains a combination of:

a) 0.1 to 25.0 weight-% betain, and
b) 0.1 to 10.0 weight-% of at least one aliphatic organic acid which is free of amino groups.

2. Hair treatment medium according to claim 1, characterised in that the betain is present in an amount from 3.0 to 15.0 weight-%.

3. Hair treatment medium according to claim 1 an 2, characterised in that the organic acid which is free from amino groups is selected from lactic acid, tartaric acid, pimelic acid, glyoxylic acid, and citric acid.

4. Hair treatment medium according to claim 1 to 3, characterised in that it is a hair rinse, a hair care emulsion, a hair fixative, or shampoo.

5. Hair treatment medium according to claim 1 to 4, characterised in that it contains

a) 3.0 to 15.0 weight-% of betain and
b) 0.1 to 5.0 weight-% of citric acid.

6. Process for the improvement of the condition of hair, characterised in that the hair is brought into contact with a hair treatment medium according to any of claims 1 to 5 at a temperature of about 15 to 60° C.

**Revendications**

1. Produit de traitement capillaire, caractérisé en ce qu'il renferme une combinaison de

a) 0,1 à 25,0% en poids de bétaine, et
b) 0,1 à 10,0% en poids d'au moins un acide organique aliphatique dépourvu de groupe amino.

2. Produit selon la revendication 1, caractérisé en ce que la proportion de bétaine qu'il renferme est comprise entre 3,0 et 15,0 en poids.

3. Produit selon les revendications 1 et 2, caractérisé en ce que l'acide organique aliphatique dépourvu de groupe amino est choisi parmi l'acide lactique, l'acide tartrique, l'acide pimélique, l'acide glyoxilique et l'acide citrique.

4. Produit selon les revendications 1 à 3, caractérisé en ce que c'est un rincage capillaire, une émulsion de soins capillaire, un fixateur des cheveux ou un shampoing.

5. Produit selon les revendications 1 à 4, caractérisé en ce qu'il renferme

a) 3,0 à 15,0% en poids de bétaine, et
b) 0,1 à 5,0% en poids d'acide citrique.

6. Procédé d'amélioration de l'état des cheveux, caractérisé en ce qu'on met les cheveux en contact avec un produit de traitement capillaire selon l'une quelconque des revendications 1 à 5, à une température d'environ 15 à 60° C.